# EUROPEAN PATENT SPECIFICATION

(11) **EP 1 286 666 B1**
(45) Date of publication and mention of the grant of the patent: **12.01.2005**
(21) Application number: 01941933.2
(22) Date of filing: 05.06.2001
(51) Int. Cl.: A61K 31/00

(54) **STARTER KIT FOR LOW DOSE ORAL CONTRACEPTIVES**
STARTERKIT VON NIEDRIGDOSIERTEM ORALEM KONTRAZEPTIV
KIT DE DEPART POUR CONTRACEPTIFS ORAUX A FAIBLE DOSE

(30) Priority: 08.06.2000 US 210310 P
(43) Date of publication of application: 05.03.2003
(73) Proprietor: Wyeth, Madison, New Jersey 07940-0874 (US)
(72) Inventor: GRUBB, Gary, S., Newtown Square, PA 19073 (US)
(74) Representative: Mannion, Sally Kim, Dr.
(86) International application number: PCT/US2001/018142
(87) International publication number: WO 2001/093848

(56) References cited:
- WO-A-97/41870
- WO-A-98/04268

## Description

### BACKGROUND OF THE INVENTION

Since the introduction of oral contraceptives (OCs) over a quarter-century ago, research has been directed toward developing preparations that minimize the potential for side effects while maintaining efficacy and normal menstrual patterns.

At the start of an OC regimen, women experience rates of breakthrough bleeding and spotting in the first OC cycle which are about two times higher than the normal rates which remain generally steady after the fourth OC cycle. This is due to the change in the histology of the endometrium over during the first three to four cycles that is due to the effect if a progestin in the OC. Several months of OC use produces a more secretory endometrium which is less prone to breakthrough bleeding. Breakthrough bleeding and spotting are the most common complaints from women starting to use OCs and is a common reason for discontinuing their use. Furthermore, breakthrough bleeding and spotting rates are higher with OCs containing amounts of estrogen less than about 30 micrograms (µg), particularly in the first several months of use.

Using a constant dose of progestin, Endrikat et al., 1997 found that the break through bleeding/spotting rate for 30µg ethinyl estradiol (EE) OC as compared to 20µg EE OC was 68% in cycle 1, 85% in cycle 2 and 67% in cycle 3. Similarly, Akerlund et al. (1993) found that the break through bleeding/spotting rate for 30µg EE OC as compared to 20µg EE OC was 77% in cycle 1, 60% in cycle 2 and 67% in cycle 3. These results are consistent with the conversion to a secretory endometrium occurring more quickly with an OC containing higher EE doses.

The present invention provides a contraceptive kit which helps to overcome or ameliorate the problem of breakthrough bleeding and spotting associated with starting oral contraception using the lowest dose (15-20 µg EE) estrogen contraceptives.

### DETAILED DESCRIPTION OF THE INVENTION

In accordance with the present invention is provided an oral contraceptive starter kit comprising at least two cycle packs of oral contraceptives containing an estrogen and a progestin, said kit having a first cycle pack and a last cycle pack, the first cycle pack containing an effective dosage of estrogen and progestin greater than the last cycle pack.

It is preferred that the last cycle pack provides the lowest amount of effective steroid dosage, and most preferably the lowest amount of estrogen. It is also preferred that the effective dosage of estrogen and progestin in the first cycle pack is greater than in subsequent cycle packs. Most preferably, the last cycle pack preferably provides no more than 20 µg of estrogen per dosage unit.

In a further embodiment, the effective dosage of estrogen and progestin in the penultimate cycle pack is greater than the effective dosage of estrogen and progestin in the last cycle pack.

The starter kit provides a means to gradually decrease the dose of estrogen over a number of cycles, thereby decreasing incidences of breakthrough bleeding and spotting that are often associated the lose-dose estrogen contraceptives. Thus, a first cycle may contain as a daily dosage, for example, 30 µg of estrogen per dosage unit. A second cycle may contain 30µg of estrogen per dosage unit. A third cycle may contain 20 µg of estrogen per dosage unit. Following the completion of the starter kit, standard cycle packs of lowest dose estrogen OC may be used.

Hormonal active ingredients useful as oral contraceptives are well known in the art. Generally oral contraceptives contain an estrogen and a progestin (steroids) and may be formulated as monophasic, biphasic or triphasic cycles.

Suitable estrogens include 17-β estradiol, estrone, or a salt thereof, estriol, ethinylestradiol and mestranol. Ethyinylestradiol (EE) is the preferred estrogen.

Suitable progestins include trimegestone, nomegestrol, dienogest, norgestrel, levonorgestrol, cyproterone acetate, 3-ketodesogestrel (or etonogestrel), desogestrel, gestodene, norethindrone, drospirenone, medroxy progesterone acetate, megestrol acetate, norgestimate, 17β deactyl norgestimate, osaterone, norethindrone acetate, lynestrenol, norethynodrel, and ethynodiol diacetate.

Combination oral contraceptives (containing estrogen and progestins) are commercially available such as those sold under the tradenames Alesse®, Brevicon®, Demulen®, Desogen®, Estrostep®, Harmonet®, Levlen®, Levlite®, Levora®, Loestrin®, Loette®, LoOvral®, Micronor®, Minesse®, Minulet®, Mircette®, Modicon®, Necon®, Nordette®, Norinyl®, Ortho-cept®, Ortho-Cyclen®, Ortho-Novum®, Ortho-Tri-Cyclen®, Ovcon®, Ovral®, Ovrette®, Tritevlen®, Trimiron®, TriMinulet®, Tri-Norinyl®, Triphasil®, Trivora®, and Zovia®.

Kits of the present invention may contain multiple cycles dosages of various combination oral contraceptive arranged to appropriately decrease the effective dosage of total steroid from the penultimate cycle pack to the last cycle pack. For instance, a kit of the present invention might combine as a first cycle Nordette®, as a second cycle Triphasil® and as a third cycle Alesse®. Alternatively, a kit of the present invention might combined first and second cycles of Nordette®, third and fourth cycles of Triphasil® and a fifth cycle of Alesse®. In another embodiment of the invention, the kit might present a first cycle of Levora®, a second cycle of Trilevora® or Trilevlen® and a third cycle of Levlite®. In yet another embodiment of the invention, a first cycle of Loestrin® 1.5/30, a second cycle of Estrostep® and a third cycle of Loestrin® 1/20 might be combined. Similarly, a first cycle of Ortho-Novum@ 1/35, a second cycle of Ortho-Novum® 7/7/7 and a third cycle of an OC containing norethindrone and an EE dose of less than or equal to 30 µg are combined in a starter kit. More detailed examples of cycles and dosages are described in the Examples.

Although not required, in some preferred embodiments of the present invention the progestin and estrogen should be the same as those used in previous cycles, although the dosages are varied throughout the cycles of the kit.

Effective dosage of estrogen and progestin refers to the combined amount of estrogen and progestin (steroids) in a daily dosage unit taking into account the potency of a given steroid. The effective dose of a given steroid can be determined by one skilled in the art.

Cycle pack, as used herein, refers to an oral contraceptive pill pack generally containing from 21-25 consecutive days of active ingredient-containing dosage units and may also contain placebos for the remainder of the cycle (3 to 7 days), which are free of hormonal active ingredient. Dosage units in the form of tablets or capsules may also contain excipients such as binders, diluents, disintegrating agents and lubricating agents. Placebos of the cycle pack may contain non-hormonal active agents such as iron or folic acid.

Each cycle preferably ranges in duration from 21-25 consecutive days of steroid, followed by non-contraceptive placebos for the remainder of each cycle (i.e. 3-7 days). The kit may contain daily dosages arranged in dispensers such as blister packs or dial pack dispensers.

The starter kit may contain 2 or more single cycle dosage arrangements, or the cycles may be combined to form a multi-cycle dosage arrangement. The starter kit may also contain instructional materials, markings or arrangements which explain the use and order of the cycle packs.

In multi-cycle dosage arrangements the cycles may be separated from one another spatially and/or by other markings. Alternatively, blister packs containing individual cycles may be separated by perforations in the base of the blister pack or other means suitable for separation.

### Examples

The following examples are illustrative the present invention. Each example describes regimens of combination oral contraceptives which can be used to decrease the incidence of break through bleeding and spotting associated with the lowest dose (15-20µg) estrogen contraceptives.

### Example 1

| **Compound** | **Amount (dosage unit)** | **Duration** | **Cycle** |
|---|---|---|---|
| LNG (levonorgestrel) | 150 µg | 21 days | 1 |
| EE (ethinyl estradiol) | 30 µg | | |
| LNG | 50 µg | 6 days | 2 |
| EE | 30 µg | | |
| LNG | 75 µg | 5 days | |
| EE | 40 µg | | |
| LNG | 125 mg | 10 days | |
| EE | 30 µg | | |
| LNG | 100 µg | 21 days | 3 |
| EE | 20 µg | | |

### Example 2

| **Compound** | **Amount (dosage unit)** | **Duration** | **Cycle** |
|---|---|---|---|
| LNG (levonorgestrel) | 150 µg | 21 days | 1, 2 |
| EE (ethinyl estradiol) | 30 µg | | |
| LNG | 50 µg | 6 days | 3, 4 |
| EE | 30 µg | | |
| LNG | 75 µg | 5 days | |
| EE . | 40 µg | | |
| LNG | 125 mg | 10 days | |
| EE | 30 µg | | |
| LNG | 100 µg | 21 days | 5 |
| EE | 20 µg | | |

### Example 3

| **Compound** | **Amount** | **Duration** | **Cycle** |
|---|---|---|---|
| NETA (norethindrone acetate) | 1.5 mg | 21 days | 1 |
| EE | 30 µg | | |
| NETA | 1 mg | 5 days | 2 |
| EE | 20 µg | | |
| NETA | 1 mg | 7 days | |
| EE | 30 µg | | |
| NETA | 1 mg | 9 days | |
| EE | 35 µg | | |
| NETA | 1 mg | 21 days | 3 |
| EE | 20 µg | | |

### Example 4

| **Compound** | **Amount** | **Duration** | **Cycle** |
|---|---|---|---|
| NET (norethindrone) | 1.0 mg | 21 days | 1 |
| EE | 35 µg | | |
| NET | 0.5 mg | 7 days | 2 |
| EE | 35 µg | | |
| NET | 0.75 mg | 7 days | |
| EE | 35 µg | | |
| NET | 1 mg | 7 days: | |
| EE | 35 µg | | |
| NET | 1 mg | 21 days | 3 |
| EE | 25 µg | | |

### Example 5

| **Compound** | **Amount** | **Duration** | **Cycle** |
|---|---|---|---|
| GSD (gestodene) | 75 µg | 21 days | 1 |
| EE | 30 µg | | |
| GSD | 50 µg | 6 days | 2 |
| EE | 30 µg | | |
| GSD | 70 µg | 5 days | |
| EE | 40 µg | | |
| GSD | 100 µg | 10 days | |
| EE | 30 µg | | |
| GSD | 75 µg | 21 days | 3 |
| EE | 20 µg | | |

### Example 6

| **Compound** | **Amount** | **Duration** | **Cycle** |
|---|---|---|---|
| GSD | 50 µg | 6 days | 1 |
| EE | 30 µg | | |
| GSD | 70 µg | 5 days | |
| EE | 40 µg | | |
| GSD | 100 µg | 10 days | |
| EE | 30 µg | | |
| GSD | 75 µg | 21 days | 2 |
| EE | 30 µg | | |
| GSD | 75 µg | 24 days | 3 |
| EE | 20 µg | | |

### Example 7

| **Compound** | **Amount** | **Duration** | **Cycle** |
|---|---|---|---|
| GSD | 75 µg | 21 days | 1 |
| EE | 30 µg | | |
| GSD | 75 µg | 21 days | 2 |
| EE | 20 µg | | |
| GSD | 60 µg | 24 days | 3 |
| EE | 15 µg | | |

### Example 8

| **Compound** | **Amount** | **Duration** | **Cycle** |
|---|---|---|---|
| GSD | 75 µg | 21 days | 1,2 |
| EE | 30 µg | | |
| GSD | 75 µg | 21 days | 3,4 |
| EE | 20 µg | | |
| GSD | 60 µg | 24 days | 5 |
| EE | 15 µg | | |

### Example 9

| **Compound** | **Amount** | **Duration** | **Cycle** |
|---|---|---|---|
| GSD | 75 µg | 21 days | 1 |
| EE | 30µg | | |
| GSD | 50 µg | 6 days | 2 |
| EE | 30 µg | | |
| GSD | 70 µg | 5 days | |
| EE | 40 µg | | |
| GSD | 100 µg | 10 days | |
| EE | 30 µg | | |
| GSD | 60 µg | 24 days | 3 |
| EE | 15 µg | | |

### Example 10

| **Compound** | **Amount** | **Duration** | **Cycle** |
|---|---|---|---|
| DSG | 150 µg | 21 days | 1 |
| EE | 30µg | | |
| DSG | 50 µg | 7 days | 2 |
| EE | 35 µg | | |
| DSG | 100 µg | 7 days | |
| EE | 30 µg | | |
| DSG | 150 µg | 7 days | |
| EE | 30 µg | | |
| DSG | 150 µg | 21 days | 3 |
| EE | 20 µg | | |
| EE | 10 µg | 5 days | |

### Example 11

| **Compound** | **Amount** | **Duration** | **Cycle** |
|---|---|---|---|
| NGM | 250 µg | 21 days | 1 |
| EE | 30 µg | | |
| NGM | 180 µg | 7 days | 2 |
| EE | 35 µg | | |
| NGM | 215 µg | 7 days | |
| EE | 35 µg | | |
| NGM | 250 µg | 7 days | |
| EE | 30 µg | | |
| NGM | 250 µg | 21 days | 3 |
| EE | 20µg | | |

### Example 12

| **Compound** | **Amount** | **Duration** | **Cycle** |
|---|---|---|---|
| NGM (norgestimate) | 250 µg | 21 days | 1,2 |
| EE | 30 µg | | |
| NGM | 180 µg | 7 days | 3, 4 |
| EE | 35 µg | | |
| NGM | 215 µg | 7 days | |
| EE | 35 µg | | |
| NGM | 250 µg | 7 days | |
| EE | 30 µg | | |
| NGM | 250 µg | 21 days | 5 |
| EE | 20 µg | | |

## Claims

1. An oral contraceptive starter kit comprising at least two cycle packs of oral contraceptives containing an estrogen and a progestin, wherein each cycle pack contains from 21-25 consecutive days of active ingredient containing dosage units, said kit having a first cycle pack and a last cycle pack, the first cycle pack containing a combined amount of estrogen and progestin in a daily dosage unit greater than the last cycle pack.

2. The starter kit according to claim 1 in which the last cycle pack provides a daily dosage of estrogen equivalent to 20 µg ethyinylestradiol per dosage unit.

3. The starter kit according to claim 1 in which the effective dosage of estrogen and progestin in the first cycle pack is greater than in subsequent cycle packs.

4. The starter kit according to claim 1 in which the effective dosage of estrogen and progestin in the penultimate cycle pack is greater than the effective dosage of estrogen and progestin in the last cycle pack.

5. The starter kit according to claim 1 in which the last cycle pack contains the lowest effective dosage of estrogen and progestin.

6. The starter kit according to claim 1 wherein the cycle packs are packaged in individual units.

7. The starter kit according to claim 1 wherein multiple cycle packs are packaged together as a single unit.

8. The starter kit according to claim 1 further comprising written instructions describing the order of use of said cycle packs.

9. The starter kit according any of the preceding claims wherein the estrogen is ethinylestradiol.

10. The starter kit according to any of the preceding claims wherein the progestin is selected from the group consisting of trimegestone, nomegestrol, norgestrel, levonorgestrol, cyproterone acetate, 3-ketodesogestrel, desogestrel, gestodene, drospirenone, medroxy progesterone acetate, megestrol acetate, norgestimate, 17B deactyl norgestimate, osaterone, norethindrone, norethindrone acetate, lynestrenol, norethynodrel, and ethynodiol diacetate.

11. The starter kit according to any of the preceding claims wherein the estrogen is ethinylestradiol and the progestin is levonorgestrel.

12. The starter kit according to any of the preceding claims wherein the estrogen is ethinylestradiol and the progestin is norethindrone.

13. The starter kit according to any of the preceding claims wherein the estrogen is ethinylestradiol and the progestin is norethindrone acetate.

14. The starter kit according to any of the preceding claims wherein the estrogen is ethinylestradiol and the progestin is gestodene.

15. The starter kit according to any of the preceding claims wherein the estrogen is ethinylestradiol and the progestin is norgestimate.

## Patentansprüche

1. Orales Empfängnisverhütungsmittel-Starter-Kit, welches mindestens zwei Zykluspackungen an oralen Empfängnisverhütungsmitteln umfasst, welche ein Estrogen und ein Progestin enthalten, wobei jede Zykluspackung von 21-25 fortlaufende Tage an Wirkstoff enthaltenden Dosierungseinheiten enthält, besagtes Kit eine erste Zykluspackung und eine letzte Zykluspackung hat, die erste Zykluspackung eine vereinigte Menge an Estrogen und Progestin in einer täglichen Dosierungseinheit enthält, welche größer ist, als die letzte Zykluspackung.

2. Starter-Kit gemäß Anspruch 1, worin die letzte Zykluspackung eine tägliche Dosierung an Estrogen vorsieht, welche zu 20 *µ*g Ethinylestradiol pro Dosierungseinheit äquivalent ist.

3. Starter-Kit gemäß Anspruch 1, worin die wirksame Dosierung an Estrogen und Progestin in der ersten Zykluspackung größer ist, als in den nachfolgenden Zykluspackungen.

4. Starter-Kit gemäß Anspruch 1, worin die wirksame Dosierung an Estrogen und Progestin in der vorletzten Zykluspackung größer ist, als die wirksame Dosierung an Estrogen und Progestin in der letzten Zykluspackung.

5. Starter-Kit gemäß Anspruch 1, worin die letzte Zykluspackung die niedrigste wirksame Dosierung an Estrogen und Progestin enthält.

6. Starter-Kit gemäß Anspruch 1, worin die Zykluspackungen in einzelnen Einheiten verpackt sind.

7. Starter-Kit gemäß Anspruch 1, worin multiple Zykluspackungen zusammen als einzelne Einheit verpackt sind.

8. Starter-Kit gemäß Anspruch 1, welches ferner schriftliche Anweisungen enthält, welche die Reihenfolge der Verwendung von besagten Zykluspackungen beschreiben.

9. Starter-Kit gemäß einem der vorhergehenden Ansprüche, worin das Estrogen Ethinylestradiol ist.

10. Starter-Kit gemäß einem der vorhergehenden Ansprüche, worin das Progestin ausgewählt wird aus der Gruppe bestehend aus Trimegeston, Nomegestrol, Norgestrel, Levonorgestrol, Cyproteronacetat, 3-Ketodesogestrel, Desogestrel, Gestoden, Drospirenon, Medroxyprogesteronacetat, Megestrolacetat, Norgestimat, 17B-Deactylnorgestimat, Osateron, Norethindron, Norethindronacetat, Lynestrenol, Norethynodrel und Ethynodioldiacetat.

11. Starter-Kit gemäß einem der vorhergehenden Ansprüche, worin das Estrogen Ethinylestradiol ist und das Progestin Levonorgestrel ist.

12. Starter-Kit gemäß einem der vorhergehenden Ansprüche, worin das Estrogen Ethinylestradiol ist und das Progestin Norethindron ist.

13. Starter-Kit gemäß einem der vorhergehenden Ansprüche, worin das Estrogen Ethinylestradiol und das Progestin Norethiridronacetat ist.

14. Starter-Kit gemäß einem der vorhergehenden Ansprüche, worin das Estrogen Ethinylestradiol und das Progestin Gestoden ist.

15. Starter-Kit gemäß einem der vorhergehenden Ansprüche, worin das Estrogen Ethinylestradiol ist und das Progestin Norgestimat ist.

## Revendications

1. Kit de départ de contraceptif oral comprenant au moins deux emballages pour cycle de contraceptifs oraux contenant un oestrogène et une progestine, dans lequel chaque emballage pour cycle contient des unités de dosage contenant des ingrédients actifs pour entre 21 et 25 jours consécutifs, ledit kit ayant un premier emballage pour cycle et un dernier emballage pour cycle, le premier emballage pour cycle contenant une quantité combinée d'oestrogène et de progestine dans une unité de dosage quotidienne supérieure à celle du dernier emballage pour cycle.

2. Kit de départ selon la revendication 1, dans lequel le dernier emballage pour cycle fournit un dosage quotidien d'oestrogène équivalent à 20 µg d'éthinylestradiol par unité de dosage.

3. Kit de départ selon la revendication 1, dans lequel le dosage efficace d'oestrogène et de progestine dans le premier emballage pour cycle est supérieur à celui des emballages pour cycle subséquents.

4. Kit de départ selon la revendication 1, dans lequel le dosage efficace d'oestrogène et de progestine dans l'avant-dernier emballage pour cycle est supérieur à celui du dosage efficace d'oestrogène et de progestine dans le dernier emballage pour cycle.

5. Kit de départ selon la revendication 1, dans lequel le dernier emballage pour cycle contient le dosage efficace d' oestrogène et de progestine le plus faible.

6. Kit de départ selon la revendication 1, dans lequel les emballages pour cycle sont emballés dans des unités individuelles.

7. Kit de départ selon la revendication 1, dans lequel les emballages pour cycle multiples sont emballés ensemble sous la forme d'une unité individuelle.

8. Kit de départ selon la revendication 1, comprenant en outre des instructions écrites décrivant l'ordre d'utilisation desdits emballages pour cycle.

9. Kit de départ selon l'une quelconque des revendications précédentes, dans lequel l'oestrogène est l'éthinylestradiol.

10. Kit de départ selon l'une quelconque des revendications précédentes, dans lequel la progestine est choisie dans le groupe composé de la trimégestone, du nomégestrol, du norgestrel, du lévonorgestrol, de l'acétate de cyprotérone, du 3-cétodésogestrel, du désogestrel, du gestodène, de la drospirénone, de l'acétate de médroxyprogestérone, de l'acétate de mégestrol, du norgestimate, du norgestimate de 17β-déactyle, de l'osatérone, de la noréthindrone, de l'acétate de noréthindrone, du lynestrénol, du noréthynodrel et du diacétate d'éthynodiol.

11. Kit de départ selon l'une quelconque des revendications précédentes, dans lequel l'oestrogène est l'éthinylestradiol et la progestine est le lévonorgestrel.

12. Kit de départ selon l'une quelconque des revendications précédentes, dans lequel l' oestrogène, est l'éthinylestradiol et la progestine est la noréthindrone.

13. Kit de départ selon l'une quelconque des revendications précédentes, dans lequel l'oestrogène est l'éthinylestradiol et la progestine est l'acétate de noréthindrone.

14. Kit de départ selon l'une quelconque des revendications précédentes, dans lequel l'oestrogène est l'éthinylestradiol et la progestine est le gestodène.

15. Kit de départ selon l'une quelconque des revendications précédentes, dans lequel l'oestrogène est l'éthinylestradiol et la progestine est le norgestimate.
